# EUROPEAN PATENT APPLICATION

(11) **EP 4 471 133 A1**
(43) Date of publication of application: **04.12.2024**
(21) Application number: 22922953.9
(22) Date of filing: 30.01.2022
(51) Int. Cl.: C12N 9/00, C12Q 1/6844

(54) **IMPROVED ENZYME PELLET, AND PREPARATION METHOD THEREFOR AND USE THEREOF**

(71) Applicant: Oxford University (Suzhou) Science & Technology Co., Ltd., Suzhou, Jiangsu 215123 (CN)
(72) Inventor: YEUNG, Danny Sheng Wu, Hong Kong 999077 (CN); MA, Wu-Po, Hong Kong 999077 (CN); HSU, Chia-Chen, Hong Kong 999077 (CN)
(74) Representative: Hepp Wenger Ryffel AG
(86) International application number: PCT/CN2022/075265
(87) International publication number: WO 2023/142129

(57) **Abstract**

The present disclosure provides an enzyme composition and a preparation method therefor and a use thereof. The enzyme composition includes an enzyme, an enzyme stabilizer, and a filler. The specific enzyme stabilizer and filler are added, so that the enzyme composition can be preserved at room temperature after lyophilization and can maintain good stability and activity. Therefore, the product has improved adaptability to an ambient temperature, and is suitable for large-scale production. The enzyme composition is applied to target nucleic acid detection to maintain high sensitivity and detection stability.

## Description

### TECHNICAL FIELD

The present disclosure relates to the field of biochemical reagents. Specifically, the present disclosure relates to an improved enzyme pellet and a preparation method therefor and a use thereof.

### BACKGROUND

A DNA polymerase is a type of enzyme for biologically catalyzing a deoxyribonucleic acid (DNA) and a ribonucleic acid (RNA), is a necessary enzyme complex in a DNA biosynthesis process, and has catalytic activity for DNA synthesis and the complementary activity. With development of molecular biology, a polymerase chain reaction (PCR) technology was publicly introduced by Mullis of the United States in 1985 and has been rapidly developed. Currently, the polymerase chain reaction technology has become an extremely important technological means in the fields of molecular biology, life sciences, and the like, and is widely applied in the biological fields such as gene amplification, gene cloning, gene modification, analysis of infectious disease sources, and genetic fingerprint identification. As one of important factors of the PCR technology, the DNA polymerase plays an important role in a PCR process. In a sense, the PCR technology is a DNA polymerase technology. Currently, discovery of a thermostable DNA polymerase enables the PCR technology to be much more convenient, and greatly reduces costs of the PCR technology, thereby promoting development and application of the PCR technology. However, currently, to maintain the activity of the DNA polymerase for a long time, preservation and transportation of the DNA polymerase still need to be performed at -20°C, resulting in high costs, inconvenient operation and transportation, and varying degrees of reduction in activity of the enzyme. In recent years, there are many reports on researches of stability and activity of other enzymes during preservation and transportation, but there are few reports on a polymerase.

In 2000, a Japanese scientist Notomi published a new isothermal nucleic acid amplification technology suitable for gene diagnosis in the journal Nucleic Acids Res, that is, loop-mediated isothermal amplification, which is referred to as a "LAMP" technology for short. The "LAMP" technology is to design four or six specific primers for a target gene, and perform isothermal amplification at 60°C to 65°C under the action of a strand displacement DNA polymerase (Bst DNA Polymerase) for 15 minutes to 60 minutes, to implement 109-1010-fold nucleic acid amplification.

Compared with conventional PCR, the "LAMP" technology has the following advantages:
1. Sensitivity is high, and is two to five orders of magnitude higher than that of a conventional nucleic acid amplification method.
2. A reaction time is short, there is no need to perform thermal denaturation on a template, and a reaction can be completed within 30 minutes to 60 minutes.
3. An entire reaction process is thermostatic without temperature cycling and without relying on an expensive professional detection device in clinical use.
4. A reaction result may be determined by observing a color developer visually, there is no need to perform electrophoresis and ultraviolet observation, the reaction result is determined simply and fast, and an ordinary person who does not have a professional skill can perform quick diagnosis in a disease scene.

In recent years, the "LAMP" technology has been widely applied in many countries to quickly detect pathogenic microorganisms such as SARS, influenza virus, mycoplasma pneumoniae, and mycobacterium tuberculosis. However, in actual application, a polymerase required for a LAMP reaction needs to be preserved and transported at a low temperature below -20°C for a long time, resulting in high costs, inconvenient operation and transportation, and varying degrees of reduction in activity of the enzyme.

Therefore, it is urgently necessary to develop a polymerase capable of being preserved at room temperature, in particular, a polymerase capable of being preserved at room temperature for a LAMP reaction.

### SUMMARY

The present disclosure is intended to resolve, at least to some extent, one of technical problems in the related art. Therefore, an objective of the present disclosure is to provide an enzyme composition. The enzyme composition can be preserved at room temperature and can maintain good stability and activity. Therefore, adaptability of the enzyme composition to an ambient temperature is improved, and the enzyme composition is suitable for large-scale production. The enzyme composition is applied to target nucleic acid detection to maintain high sensitivity and detection stability.

In one aspect of the present disclosure, the present disclosure provides an enzyme composition. According to an embodiment of the present disclosure, the enzyme composition is suitable for preservation at room temperature. The enzyme composition includes:
an enzyme;
an enzyme stabilizer; and
a filler.

The enzyme stabilizer is selected from optional sucrose and at least one of trehalose or trehalose dihydrate.

The filler is selected from at least one of dextran, mannitol, polyethylene glycol, bovine serum albumin, polyvinyl alcohol, raffinose, or sorbitol.

Since the outbreak of SARS-CoV-2, nucleic acid detection (for example, qPCR or LAMP detection) becomes a main way to detect a positive or negative sample virus. To maintain activity of a DNA polymerase during preservation and transportation, cold-chain transportation, which means to maintain a preservation or transportation temperature at -20 degrees Celsius, is an existing manner used on a large scale. However, cold-chain transportation increases preservation and transportation costs and is complicated in operation. The inventors find that by adding special enzyme stabilizer and filler, for example, a trehalose dihydrate enzyme stabilizer and a dextran filler, to a liquid enzyme preparation, and lyophilizing the liquid enzyme preparation to prepare a lyophilized product, the enzyme can be preserved at room temperature, and activity and stability of the enzyme can be maintained. Therefore, the product can withstand environmental instability, can be preserved at room temperature for 1 to 7 days, and can be produced on a large scale.

According to an embodiment of the present disclosure, a weight ratio of the enzyme stabilizer to the enzyme is (5-20): 100, and a weight ratio of the filler to the enzyme is (1-15): 100. Therefore, the activity of the enzyme can be maintained and the enzyme composition can be maintained in a stable form at room temperature.

According to an embodiment of the present disclosure, the enzyme composition is a lyophilized product obtained through lyophilization.

According to an embodiment of the present disclosure, the lyophilized product is lyophilized powder or a lyophilized pellet.

According to an embodiment of the present disclosure, the enzyme composition is a pellet, and a diameter of the pellet is 0.1 mm to 20 mm.

According to an embodiment of the present disclosure, the enzyme in the enzyme composition includes at least one of a DNA polymerase, an RNA polymerase, a reverse transcriptase, a restriction endonuclease, a recombinase, a ligase, a hydrolase, a helicase, a cellulase, a pectinase, a protease, or a lipase.

According to an embodiment of the present disclosure, the enzyme in the enzyme composition includes at least one of the DNA polymerase, the RNA polymerase, the reverse transcriptase, or the recombinase.

In another aspect of the present disclosure, the present disclosure provides a method for preparing the foregoing enzyme composition. According to an embodiment of the present disclosure, the method includes:
(1) preparing an enzyme-containing preparation;
(2) mixing the enzyme-containing preparation with the enzyme stabilizer and the filler to obtain a to-be-lyophilized mixture; and
(3) lyophilizing the to-be-lyophilized mixture to obtain the enzyme composition capable of being preserved at room temperature.

According to an embodiment of the present disclosure, an enzyme in the enzyme-containing preparation is a mixture of a DNA polymerase and a reverse transcriptase.

According to an embodiment of the present disclosure, the enzyme stabilizer is trehalose dihydrate.

According to an embodiment of the present disclosure, the filler is dextran.

According to an embodiment of the present disclosure, a concentration of the enzyme is 0.1 U/µL to 5.0 U/µL.

According to an embodiment of the present disclosure, a weight ratio of the enzyme stabilizer to the enzyme is (5-20): 100.

According to an embodiment of the present disclosure, a weight ratio of the filler to the enzyme is (1-15): 100.

According to an embodiment of the present disclosure, a concentration of the enzyme stabilizer is 0.05 mg/µL to 0.2 mg/µL.

According to an embodiment of the present disclosure, a concentration of the filler is 0.01 mg/µL to 0.15 mg/µL.

According to an embodiment of the present disclosure, the lyophilization is implemented by the following steps:
1) performing liquid nitrogen freezing on the to-be-lyophilized mixture to obtain a to-be-lyophilized product; and
2) performing vacuum lyophilization on the to-be-lyophilized product at -35°C to -80°C to obtain the enzyme composition capable of being preserved at room temperature.

A time for the vacuum lyophilization is at least 35 h.

According to an embodiment of the present disclosure, a temperature for the vacuum lyophilization is -35°C to -60°C.

According to an embodiment of the present disclosure, a temperature for the vacuum lyophilization is -40°C to -45°C.

When the method for preparing the enzyme composition, especially for preparing a spherical enzyme (also referred to as "enzyme pellets" or "lyobeads" is used, the specific enzyme stabilizer and filler are added to a liquid enzyme preparation, liquid nitrogen freezing is performed to form beads, and then vacuum lyophilization is further performed under a specific condition to remove water, to obtain the lyobeads that have a complete shape and good mechanical performance and can be preserved and transported at room temperature. In addition, the enzyme lyobeads have high porosity, that is, have a high volume-surface area ratio, so that rapid resolubilization is implemented.

The pellets obtained after liquid nitrogen freezing still contain a lot of water that hydrolyzes a protein during preservation and transportation. To maintain activity of the protein and maintain stability of the protein at room temperature, the inventors perform vacuum lyophilization on the pellets. Hydrogen-bond interactions between the protein and a water molecule are reduced by removing water, resulting in a change in a three-dimensional structure of the protein and denaturation. Therefore, the specific enzyme stabilizer needs to be added to reduce changes in the structure of the protein in a drying process. Water exists in the pellets in a form of an ice crystal after liquid nitrogen is added dropwise. A hole is left due to sublimation of the ice crystal in the drying process. Therefore, the inventors add the specific filler before adding liquid nitrogen dropwise to improve mechanical strength of the pellets and maintain a porous structure of the pellets, thereby preventing the pellets from being collapsed, preventing water content of a final product from being affected, and preventing a preservation period of the product from being shortened.

Because the pellets obtained after liquid nitrogen freezing contain a lot of water, some pores occur in the pellets after the lyophilized enzyme pellets obtained after vacuum lyophilization is performed on the pellets under the specific condition loss water. The pellet form is maintained by adding the specific filler, to ensure integrity and stability of the enzyme pellets at room temperature.

According to an embodiment of the present disclosure, the room temperature is 20°C to 40°C.

In still another aspect of the present disclosure, the present disclosure provides a use of the foregoing enzyme composition or the enzyme composition prepared according to the foregoing method in preparing a kit. According to an embodiment of the present disclosure, the kit is used for detecting a target nucleic acid.

According to an embodiment of the present disclosure, the enzyme contained in the enzyme composition includes at least one of a DNA polymerase, an RNA polymerase, or a reverse transcriptase.

According to an embodiment of the present disclosure, a nucleic acid is a DNA or an RNA.

In still another aspect of the present disclosure, the present disclosure provides a kit. According to an embodiment of the present disclosure, the kit contains the foregoing enzyme composition or the enzyme composition prepared according to the foregoing method.

According to an embodiment of the present disclosure, the enzyme contained in the enzyme composition includes at least one of a DNA polymerase, an RNA polymerase, or a reverse transcriptase.

In still another aspect of the present disclosure, the present disclosure provides a nucleic acid amplification reaction system. According to an embodiment of the present disclosure, the nucleic acid amplification reaction system includes the foregoing enzyme composition or the enzyme composition prepared according to the foregoing method.

According to an embodiment of the present disclosure, the nucleic acid amplification reaction system further includes a primer, dNTP, and a reaction buffer for nucleic acid amplification.

According to an embodiment of the present disclosure, the nucleic acid amplification reaction system further includes a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

According to an embodiment of the present disclosure, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

According to an embodiment of the present disclosure, the fungus is selected from at least one of yeast, mold, candida albicans, cryptococcus, aspergillus, or mucor, and the chlamydia is chlamydia trachomatis.

According to an embodiment of the present disclosure, the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

According to an embodiment of the present disclosure, the coronavirus is selected from at least one of SARS-CoV, SARS-CoV-2, SARS-CoV-19, MERS-CoV, HCoV-229E, HCoV-OC43, HCoV-NL63, or HCoV-HKU1.

According to an embodiment of the present disclosure, the influenza virus is selected from influenza A virus and influenza B virus.

According to an embodiment of the present disclosure, the protozoan is selected from at least one of plasmodium, trichomonas vaginalis, toxoplasma gondii, leishmania, babesia, entamoeba histolytica, acanthamoeba, or trypanosoma brucei.

In still another aspect of the present disclosure, the present disclosure provides a LAMP (Loop-mediated isothermal amplification) reaction system. According to an embodiment of the present disclosure, the LAMP reaction system includes the foregoing enzyme composition or the enzyme composition prepared according to the foregoing method.

The foregoing enzyme composition is applied to the LAMP reaction system, especially, a mixture of a DNA polymerase and a reverse transcriptase are prepared into lyophilized pellets, so that the enzyme does not need to be preserved at a low temperature and can be directly preserved at room temperature to maintain good stability and activity, thereby improving adaptability of the enzyme to an ambient temperature. In addition, enzyme content of the enzyme pellet may be controlled as required, so that the enzyme pellet is used in a reaction system. When sample addition is performed in a LAMP reaction, only one to more lyophilized pellets need to be added without performing repeated addition by using a pipette, thereby avoiding a problem that the enzyme is easily contaminated and inactivated when an existing liquid enzyme is added to the reaction system, shortening a time for preparing the LAMP reaction system, and improving working efficiency.

According to an embodiment of the present disclosure, the enzyme in the enzyme composition is a DNA polymerase or a mixture of a DNA polymerase and a reverse transcriptase.

According to an embodiment of the present disclosure, the DNA polymerase is a Bst DNA polymerase.

According to an embodiment of the present disclosure, the LAMP reaction system further includes a primer, dNTP, and a reaction buffer for nucleic acid amplification.

According to an embodiment of the present disclosure, the LAMP reaction system further includes a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

According to an embodiment of the present disclosure, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

According to an embodiment of the present disclosure, the fungus is selected from at least one of yeast, mold, candida albicans, cryptococcus, aspergillus, or mucor, and the chlamydia is chlamydia trachomatis.

According to an embodiment of the present disclosure, the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

According to an embodiment of the present disclosure, the coronavirus is selected from at least one of SARS-CoV, SARS-CoV-2, SARS-CoV-19, MERS-CoV, HCoV-229E, HCoV-OC43, HCoV-NL63, or HCoV-HKU1.

According to an embodiment of the present disclosure, the influenza virus is selected from influenza A virus and influenza B virus.

According to an embodiment of the present disclosure, the protozoan is selected from at least one of plasmodium, trichomonas vaginalis, toxoplasma gondii, leishmania, babesia, entamoeba histolytica, acanthamoeba, or trypanosoma brucei.

In still another aspect of the present disclosure, the present disclosure provides a method for detecting a target nucleic acid in a to-be-detected sample. According to an embodiment of the present disclosure, the method includes:
(i). obtaining the to-be-detected sample or obtaining a nucleic acid isolated from the to-be-detected sample;
(ii). using the to-be-detected sample or the nucleic acid as a template, and amplifying the template by using a specific primer for the target nucleic acid and using the foregoing enzyme composition or the enzyme composition prepared according to the foregoing method, to obtain an amplified product; and
(iii). detecting whether the amplified product contains a plurality of copies of the target nucleic acid, where if the amplified product contains the plurality of copies of the target nucleic acid, it indicates that the to-be-detected sample contains the target nucleic acid.

According to an embodiment of the present disclosure, the method further includes:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample.

According to an embodiment of the present disclosure, the reference sequence is a nucleic acid sequence of a wild-type target nucleic acid that is not mutated.

According to an embodiment of the present disclosure, the nucleic acid is a DNA or an RNA.

According to an embodiment of the present disclosure, the to-be-detected sample is derived from a mammal or an environment sample.

According to an embodiment of the present disclosure, the mammal is selected from a human, a rat, a mouse, a pig, cattle, a sheep, a dog, and a cat.

Additional aspects and advantages of the present disclosure will be partly given in the following description, and part thereof will become apparent from the following description, or may be learned through the practice of the present disclosure.

### BRIEF DESCRIPTION OF DRAWINGS

The above-mentioned and/or additional aspects and advantages of the present disclosure become apparent and easy to understand from the description of the embodiments in conjunction with the following accompanying drawings.
FIG. 1 is a flowchart of a method for preparing an enzyme composition according to an embodiment of the present disclosure;
FIG. 2 is a flowchart of a method for preparing an enzyme pellet according to an embodiment of the present disclosure;
FIG. 3 shows a form of an enzyme pellet according to the present disclosure;
FIG. 4 shows color development results of reaction products obtained after an enzyme preparation and an enzyme pellet in the present disclosure are separately applied to a LAMP reaction according to Embodiment 3 of the present disclosure;
FIG. 5 shows color development results of reaction products obtained after a lyophilized product and an enzyme pellet are separately applied to a LAMP reaction according to Embodiment 4 of the present disclosure;
FIG. 6 shows color development conditions of groups with different sugar concentrations in a lyophilized LAMP amplification reaction according to Embodiment 5 of the present disclosure; and
FIG. 7 shows color development conditions of sample tubes before and after a LAMP amplification reaction in which enzyme pellets preserved for 7 days at 4°C, 25°C, 40°C, and 60°C are used according to Embodiment 6 of the present disclosure.

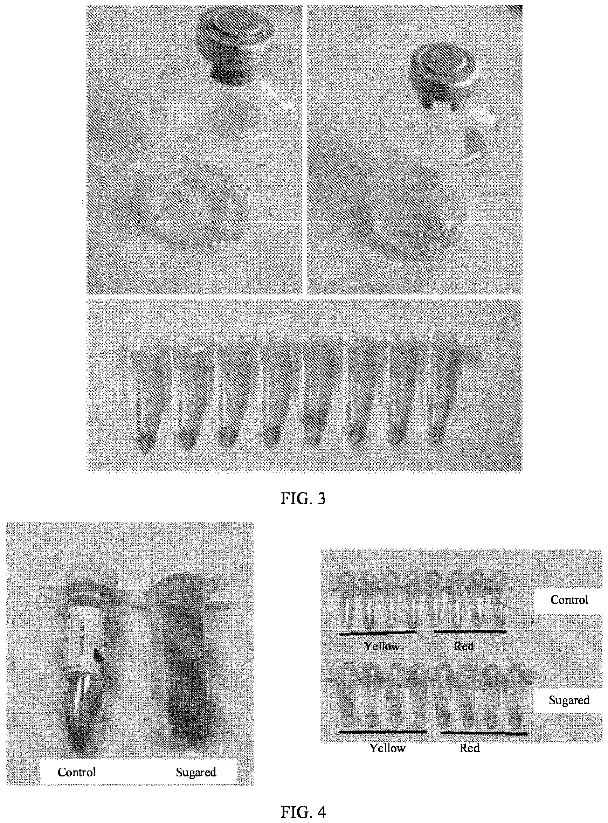

It should be noted that only some LAMP reaction results are shown in FIG. 4 to FIG. 7 in the present disclosure. Actually, the inventors perform at least three repeated tests, and obtain a same result.

### DESCRIPTION OF EMBODIMENTS

The following describes in detail the embodiments of the present disclosure. The embodiments described below are exemplary and are merely intended to explain the present disclosure and are not understood as limiting the present disclosure. If a specific technology or condition is not specified in the embodiments, a technology or condition described in the literatures in the art or a product specification is used. Reagents or instruments used without specified manufacturers are all conventional products that can be obtained from markets.

In the description of the present disclosure, "a plurality of" means at least two, for example, two or three, unless otherwise specifically limited.

### Enzyme Composition

In an implementation of the present disclosure, the present disclosure provides an enzyme composition. The enzyme composition includes an enzyme, an enzyme stabilizer, and a filler. The enzyme stabilizer is selected from optional sucrose and at least one of trehalose or trehalose dihydrate. The filler is selected from at least one of dextran, mannitol, polyethylene glycol, bovine serum albumin, polyvinyl alcohol, raffinose, or sorbitol.

In an implementation of the present disclosure, a weight ratio of the enzyme stabilizer to the enzyme is (5-20): 100, and a weight ratio of the filler to the enzyme is (1-15):100.

Activity of the enzyme can be maintained and a stable form of the enzyme composition can be maintained at room temperature when the weight ratio of the enzyme stabilizer to the filler falls within this range. With regard to content of the enzyme in the enzyme composition, an amount of enzyme commonly used by a person skilled in the art may be added to a liquid enzyme preparation (also referred to as an "enzyme-containing preparation" herein) during preparation of the enzyme composition based on different types of enzymes in the formed enzyme composition and different uses of the enzymes. For example, if the enzyme in the enzyme composition is used for a LAMP reaction, the enzyme contained in the enzyme composition is a mixture of a DNA polymerase and a reverse transcriptase, and a corresponding amount of enzyme is added based on a concentration of a liquid enzyme preparation used for the LAMP reaction. Based on this, the enzyme stabilizer and the filler are added, and lyophilization is performed after mixing.

For example, the enzyme contained in the enzyme composition is a mixture of a Bst DNA polymerase and the reverse transcriptase when the enzyme in the enzyme composition is used for the LAMP reaction, where the weight ratio of the enzyme stabilizer to the enzyme is (5-20): 100, and the weight ratio of the filler to the enzyme is (1-15):100.

In an implementation of the present disclosure, the enzyme composition is a lyophilized product obtained through lyophilization, and the lyophilized product is lyophilized powder or a lyophilized pellet. A form of the lyophilized product is not limited herein. A person skilled in the art may correspondingly adjust the form of the lyophilized product as required by controlling a lyophilization process.

In an implementation of the present disclosure, the enzyme composition is a pellet, and a diameter of the pellet is 0.1 mm to 20 mm (for example, the diameter may be 0.1 mm, 0.2 mm, 0.3 mm, 0.4 mm, 0.5 mm, 0.6 mm, 0.7 mm, 0.8 mm, 0.9 mm, 1.5 mm, 2.0 mm, 2.5 mm, 3.0 mm, 3.5 mm, 4.0 mm, 4.5 mm, 5.0 mm, 5.5 mm, 6.0 mm, 6.5 mm, 7.0 mm, 7.5 mm, 8.0 mm, 8.5 mm, 9.0 mm, 9.5 mm, 10 mm, 11 mm, 12 mm, 13 mm, 14 mm, 15 mm, 16 mm, 17 mm, 18 mm, 19 mm, or 12 mm). When the enzyme exists in a pellet state (also referred to herein as an "enzyme pellet" or a "lyophilized pellet"), and the enzyme needs to be added to a reaction system, the enzyme pellet can be added directly without using a pipette for a plurality times as adding the liquid enzyme preparation, thereby shortening a time of an operator and improving efficiency.

In an implementation of the present disclosure, a type of the enzyme in the enzyme composition is not limited, for example, may be a DNA polymerase, an RNA polymerase, a reverse transcriptase, a restriction endonuclease, a recombinase, a ligase, a hydrolase, a helicase, a cellulase, a pectinase, a protease, or a lipase. Alternatively, the enzyme may be another enzyme commonly used in the art, provided that the enzyme is prepared into the enzyme composition in the present disclosure, so that the enzyme can be preserved and transported at room temperature and maintain high activity and good stability.

### Preparation of an Enzyme Composition

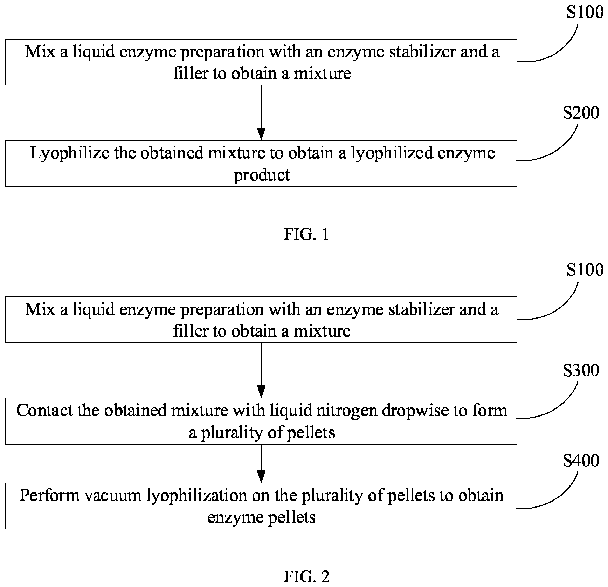

In an implementation of the present disclosure, the present disclosure provides a method for preparing the foregoing enzyme composition. As shown in FIG. 1, the method includes the following steps.

S100: Mix a liquid enzyme preparation with the enzyme stabilizer and the filler to obtain a mixture.

S200: Lyophilize the obtained mixture to obtain a lyophilized product.

In an implementation of the present disclosure, the enzyme is a mixture of a DNA polymerase and a reverse transcriptase.

In an implementation of the present disclosure, the enzyme stabilizer is selected from optional sucrose and at least one of trehalose or trehalose dihydrate.

The enzyme stabilizer may be separate trehalose dihydrate, separate trehalose, a mixture of trehalose dihydrate and sucrose, a mixture of trehalose and sucrose, or a mixture of the three sugars, all of which have a function of stabilizing activity of the enzyme.

In an implementation of the present disclosure, the enzyme stabilizer is trehalose dihydrate.

In an implementation of the present disclosure, the filler is selected from at least one of dextran, mannitol, polyethylene glycol, bovine serum albumin, polyvinyl alcohol, raffinose, or sorbitol.

In an implementation of the present disclosure, the filler is dextran.

In an implementation of the present disclosure, when the enzyme is a mixture of a DNA polymerase and a reverse transcriptase, a concentration of the enzyme is 0.1 U/µL to 5.0 U/µL. For example, the concentration of the enzyme may be 0.1 U/µL, 0.2 U/µL, 0.3 U/µL, 0.4 U/µL, 0.5 U/µL, 0.6 U/µL, 0.7 U/µL, 0.8 U/µL, 0.9 U/µL, 1.0 U/µL, 1.5 U/µL, 2.0 U/µL, 2.5 U/µL, 3.0 U/µL, 3.5 U/µL, 4.0 U/µL, 4.5 U/µL, or 5.0 U/µL.

In an implementation of the present disclosure, a weight ratio of the enzyme stabilizer to the enzyme is (5-20): 100.

In an implementation of the present disclosure, a weight ratio of the filler to the enzyme is (1-15):100. In an implementation of the present disclosure, a concentration of the enzyme stabilizer is 0.05 mg/µL to 0.2 mg/µL. For example, the concentration of the enzyme stabilizer may be 0.05 mg/µL, 0.06 mg/µL, 0.07 mg/µL, 0.08 mg/µL, 0.09 mg/µL, 0.10 mg/µL, 0.11 mg/µL, 0.12 mg/µL, 0.13 mg/µL, 0.14 mg/µL, 0.15 mg/µL, 0.16 mg/µL, 0.17 mg/µL, 0.18 mg/µL, 0.19 mg/µL, or 0.20 mg/µL.

In an implementation of the present disclosure, a concentration of the filler is 0.01 mg/µL to 0.15 mg/µL. For example, the concentration of the filler may be 0.01 mg/µL, 0.02 mg/µL, 0.03 mg/µL, 0.04 mg/µL, 0.05 mg/µL, 0.06 mg/µL, 0.07 mg/µL, 0.08 mg/µL, 0.09 mg/µL, 0.10 mg/µL, 0.11 mg/µL, 0.12 mg/µL, 0.13 mg/µL, 0.14 mg/µL, or 0.15 mg/µL.

In an implementation of the present disclosure, based on a total weight of a to-be-lyophilized mixture, a mass percentage of the enzyme stabilizer is 5 wt% to 20 wt%, preferably, 5 wt% to 15 wt%.

In an implementation of the present disclosure, based on the total weight of the to-be-lyophilized mixture, a mass percentage of the filler is 1 wt% to 9 wt%, preferably, 1 wt% to 5 wt%.

In another implementation of the present disclosure, the present disclosure provides a method for preparing the foregoing enzyme composition. As shown in FIG. 2, the method includes the following steps.

S100: Mix a liquid enzyme preparation with the enzyme stabilizer and the filler to obtain a mixture.

S300: Contact the obtained mixture with liquid nitrogen dropwise to form a plurality of pellets.

S400: Perform vacuum lyophilization on the plurality of pellets to obtain enzyme pellets.

In an implementation of the present disclosure, a temperature for the vacuum lyophilization is -35°C to 80°C, preferably, -35°C to 60°C (for example, -35°C, -40°C, -45°C, -50°C, -55°C, or -60°C), further preferably, -40°C to -45°C (for example, -40°C, -41°C, -42°C, -43°C, -44°C, or -45°C), and a time for the vacuum lyophilization is at least 35 h.

The obtained mixture of the liquid enzyme preparation, the enzyme stabilizer, and the filler is in contact with liquid nitrogen dropwise to obtain the plurality of pellets. Because the pellets contain a lot of water in this case, the vacuum lyophilization is performed on the pellets under specific temperature and time conditions to obtain lyophilized enzyme pellets. Because some pores occur in the enzyme pellets after the enzyme pellets loss water, the specific filler is added to maintain a form of the pellets. Therefore, integrity and stability of the enzyme pellets at room temperature are ensured.

### Use of an Enzyme Composition in Preparing a Kit

In an implementation of the present disclosure, the foregoing enzyme composition or the enzyme composition prepared according to the foregoing method can be used for preparing a kit, and the kit is used for detecting a target nucleic acid. The foregoing enzyme composition can be used as a part of the kit for detecting the target nucleic acid, and is used in target nucleic acid detection. For example, the foregoing enzyme composition (for example, the enzyme pellets) may be used as a part of a LAMP nucleic acid detection kit to perform a nucleic acid amplification reaction by using the kit, to detect whether a nucleic acid obtained through amplification of a sample is the target nucleic acid.

In an implementation of the present disclosure, the enzyme contained in the enzyme composition is selected from at least one of a DNA polymerase, an RNA polymerase, or a reverse transcriptase. The enzyme contained in the enzyme composition is not limited thereto. An enzyme composition containing a different enzyme may be prepared according to different requirements of a person skilled in the art. For example, the enzyme contained in the enzyme composition may be a mixture of a Bst DNA polymerase and a reverse transcriptase when the kit is used for a LAMP nucleic acid amplification reaction.

### Kit

In an implementation of the present disclosure, the present disclosure provides a kit. The kit contains the foregoing enzyme composition or the enzyme composition prepared according to the foregoing method. The kit may also contain other components according to different detection requirements. For example, if the kit is used for a LAMP nucleic acid amplification reaction, in addition to the enzyme composition, the kit may further contain saline ions (for example, magnesium ions), a buffer, a primer, or the like.

### Nucleic Acid Amplification Reaction System

In an implementation of the present disclosure, the present disclosure provides a nucleic acid amplification reaction system. The nucleic acid amplification reaction system includes the foregoing enzyme composition or the enzyme composition prepared according to the foregoing method. A nucleic acid may be a DNA or an RNA. The nucleic acid amplification reaction system may further include a primer, dNTP, and a reaction buffer for nucleic acid amplification.

In an implementation of the present disclosure, the nucleic acid amplification reaction system provided in the present disclosure is used to perform nucleic acid amplification on a to-be-detected sample. The to-be-detected sample may contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan. For example, the nucleic acid amplification reaction system may be used to directly perform LAMP on the bacterial cells or the culture fluid, or perform LAMP by using the DNA or the RNA as a template.

In an implementation of the present disclosure, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis. The fungus is selected from at least one of yeast, mold, candida albicans, cryptococcus, aspergillus, or mucor, and the chlamydia is chlamydia trachomatis. The virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus. The coronavirus is selected from at least one of SARS-CoV, SARS-CoV-2, SARS-CoV-19, MERS-CoV, HCoV-229E, HCoV-OC43, HCoV-NL63, or HCoV-HKU1. The influenza virus is selected from influenza A virus and influenza B virus. The protozoan is selected from at least one of plasmodium, trichomonas vaginalis, toxoplasma gondii, leishmania, babesia, entamoeba histolytica, acanthamoeba, or trypanosoma brucei.

### LAMP Reaction System

In an implementation of the present disclosure, the present disclosure provides a LAMP reaction system. The LAMP reaction system includes the foregoing enzyme composition or the enzyme composition prepared according to the foregoing method.

In an implementation of the present disclosure, the enzyme in the enzyme composition is a DNA polymerase or a mixture of a DNA polymerase and a reverse transcriptase. The enzyme in the enzyme composition is a mixture of a DNA polymerase and a reverse transcriptase if a nucleic acid contained in a to-be-detected sample is an RNA. The enzyme in the enzyme composition is a DNA polymerase if a nucleic acid contained in a to-be-detected sample is a DNA.

In an implementation of the present disclosure, the DNA polymerase is a Bst DNA polymerase.

In an implementation of the present disclosure, the LAMP reaction system further includes a primer, dNTP, and a reaction buffer for nucleic acid amplification.

In an implementation of the present disclosure, the LAMP reaction system provided in the present disclosure is used to perform nucleic acid amplification on a to-be-detected sample. The to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan. For example, the LAMP reaction system may be used to directly perform LAMP on the bacterial cells or the culture fluid, or perform LAMP by using the DNA or the RNA as a template.

In an implementation of the present disclosure, the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis. The fungus is selected from at least one of yeast, mold, candida albicans, cryptococcus, aspergillus, or mucor, and the chlamydia is chlamydia trachomatis. The virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus. The coronavirus is selected from at least one of SARS-CoV, SARS-CoV-2, SARS-CoV-19, MERS-CoV, HCoV-229E, HCoV-OC43, HCoV-NL63, or HCoV-HKU1. The influenza virus is selected from influenza A virus and influenza B virus. The protozoan is selected from at least one of plasmodium, trichomonas vaginalis, toxoplasma gondii, leishmania, babesia, entamoeba histolytica, acanthamoeba, or trypanosoma brucei.

### Method for Detecting a Target Nucleic Acid in a To-Be-Detected Sample

In an implementation of the present disclosure, the present disclosure provides a method for detecting a target nucleic acid in a to-be-detected sample. The method includes the following steps.
(i). Obtain the to-be-detected sample or obtain a nucleic acid isolated from the to-be-detected sample.
(ii). Use the to-be-detected sample or the nucleic acid as a template, and amplify the template by using a specific primer for the target nucleic acid and using the foregoing enzyme composition or the enzyme composition prepared according to the foregoing method, to obtain an amplified product.
(iii). Detect whether the amplified product contains a plurality of copies of the target nucleic acid, where if the amplified product contains the plurality of copies of the target nucleic acid, it indicates that the to-be-detected sample contains the target nucleic acid.

In an implementation of the present disclosure, the method further includes:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample. The reference sequence is a nucleic acid sequence of a wild-type target nucleic acid that is not mutated.

In an implementation of the present disclosure, the nucleic acid is a DNA or an RNA.

In an implementation of the present disclosure, the to-be-detected sample is derived from a plant, an animal, a mammal, or an environmental sample.

A source of the to-be-detected sample is not limited thereto, and may alternatively be another species that requires nucleic acid detection.

In an implementation of the present disclosure, the mammal is selected from a human, a rat, a mouse, a pig, cattle, a sheep, a dog, and a cat. A species of the mammal is not limited thereto, and may alternatively be another animal that requires nucleic acid detection to assess whether the mammal is infected with a pathogenic bacterium or virus.

The following describes the solutions of the present disclosure with reference to embodiments. A person skilled in the art will understand that the following embodiments are merely intended to describe the present disclosure, and are not intended to limit the scope of the present disclosure. If a specific technology or condition is not specified in the embodiments, a technology or condition described in the literatures in the art or a product specification is used. Reagents or instruments used without specified manufacturers are all conventional products that can be obtained from markets.

### Embodiments

### Embodiment 1 Preparation of an Enzyme Pellet

### 1. Enzyme pellet preparation process

A method for preparing an enzyme pellet (lyophilized pellet) includes the following steps.
(1) Preparation of a mixture of a liquid enzyme preparation, an enzyme stabilizer, and a filler: preparing 12.5 µL of enzyme premix (containing 0.32 U/µL of Bst DNA polymerase and 0.3 U/µL of reverse transcriptase (purchased from NEB, UK, https://intemational.neb.com/products/ml800-warmstart-colorimetric-lamp-2x-master-mix-dna-rna#Product%20Information)) for a LAMP reaction, and performing homogenization through transient vortex; and adding 1.243 mg of trehalose dihydrate (Sigma-Aldrich, UK) and 1.125 mg of dextran (Sigma-Aldrich, UK) to the enzyme premix, and performing dissolution by inverting a test tube, to obtain a mixture with a total volume of 13.5 µL.
(2) Pellet preparation: loading the mixture obtained in step (1) into a dispenser; dispensing 13.5 µL of reagent to liquid nitrogen; and collecting and transferring a frozen bead to a vial, where the vial contains a thin layer of liquid nitrogen.
(3) Vacuum lyophilization: transferring the bead in step (2) to a lyophilizer (https://www.spscientific.com/Products/Freeze_Dryers_/_Lyophilizers/VirTis/Floor_ Model_Tray/Pilot_Lyophilizers/Genesis_Pilot_Lyophilizer/), where the lyophilizer is pre-cooled to 40°C; and dispensing a lyobead to a PCR tube or another IVD device after vacuum drying.

### 2. Vacuum lyophilization

A vacuum lyophilization process is as follows:
A temperature of a drying chamber in the lyophilizer is lower than -40°C before the vial in step (2) is placed in the lyophilizer. The lyophilizer is started according to the following procedure after all vials are loaded (the vials are placed on a shelf).

### 1) Heat treatment phase

**Table 1**

| Heat treatment phase | | | | |
|---|---|---|---|---|
| Step | Shelf temperature (°C) | Time (mins) | Pressure (µBar) | Description |
| The vials are loaded | -50 | N/A | Atmospheric pressure | Hold |
| 1 | -50 to -45 | 10 | 500 | Heat up (Ramp) |
| 2 | -45 | 180 | 500 | Hold |

The following parameters are set for the lyophilizer before primary drying is started.

**Table 2**

| | |
|---|---|
| Freezing temperature | -50°C |
| Additional freezing | 0 min |
| Condenser setting temperature | 60°C |
| Pressure | 500 µBar |

### 2) After relevant parameters are reached, the following drying process starts.

**Table 3**

| Primary drying | | | | |
|---|---|---|---|---|
| Step | Shelf temperature (°C) | Time (mins) | Pressure (µBar) | Description |
| 3 | -45 | 10 | 100 | Hold |
| 4 | -40 | 20 | 100 | Heat up |
| 5 | -40 | 1800 | 100 | Hold |

| Secondary drying | | | | |
|---|---|---|---|---|
| 6 | 20 | 180 | 50 | Heat up |
| 7 | 20 | 400 | 50 | Hold |

Vacuum lyophilization is performed for at least 35 h. The lyophilizer is backfilled to a pressure of 500 mBar with nitrogen after cycling. It is ensured that all the vials subjected to vacuum lyophilization are correctly covered. Then, the vials are preserved at 2°C to 8°C before use.

### Embodiment 2 Evaluation of a Size and Mechanical Performance of an Enzyme Pellet

### 1. Size of an enzyme pellet

Sizes of the enzyme pellets obtained in Embodiment 1 are measured, as shown in the following Table 4.

**Table 4**

| Sample No. | Enzyme pellet diameter (mm) |
|---|---|
| 1 | 2.60 |
| 2 | 2.68 |
| 3 | 2.60 |
| 4 | 2.73 |
| 5 | 2.61 |
| 6 | 2.76 |
| 7 | 2.78 |
| Average value | 2.68 |
| SD | 0.078 |

The foregoing results indicate that the diameters of the enzyme pellets prepared according to the method in Embodiment 1 are about 2.7 mm. In addition, a small difference between the diameters of the samples indicates that the sizes of the enzyme pellets prepared according to the method in the present disclosure are uniform. FIG. 3 shows specific forms of the enzyme pellets.

### 2. Residual water content measurement

Residual water content of the enzyme pellets obtained in Embodiment 1 is measured, and results are shown in the following Table 5.

**Table 5**

| Sample | Residual water (%) | Average value (%) |
|---|---|---|
| 1 | 0.47 | 0.39 |
| 2 | 0.39 | |
| 3 | 0.32 | |

The foregoing results indicate that the enzyme pellets prepared according to the method in Embodiment 1 contain less residual water, and an effect of vacuum lyophilization is good.

### 3. Mechanical performance evaluation

Young's Modulus represents hardness of a solid material. Higher Young's Modulus indicates a smaller possibility of changing a shape due to a mechanical load and larger mechanical hardness of the enzyme pellets. Fracture stress refers to a point at which a solid structure is broken because a load is applied to a solid surface. A solid with a small pore often has a high value.

Young's modulus and fracture stress of the enzyme pellets obtained in Embodiment 1 are measured (for a measurement method, refer to https://biopharma.co.uk/intelligent-freeze-drying/intelligent-freeze-drying/micropress/ #:~:text=The%20MicroPress%20uses%20a%201inear,for%20further%20interpretatio n%20and%20analysis.). Results are shown in the following Table 6.

**Table 6**

| Sample | Young's modulus (kPa) | Average Young's modulus (kPa) | Fracture stress (kPa) | Average value |
|---|---|---|---|---|
| 1 | 17.235 | 21.643 | N/A | N/A |
| 2 | 23.5722 | | N/A | |
| 3 | 24.122 | | N/A | |

Because the enzyme pellets are not fractured when the load is applied to the enzyme pellets, no fracture stress value is recorded in Table 6. The foregoing results indicate that the enzyme pellets prepared according to the method in the present disclosure are extremely robust and are not fractured.

### Embodiment 3 Addition of an Enzyme Stabilizer and a Filler without Affecting Activity of an Enzyme

1.243 mg of trehalose dihydrate and 1.125 mg of dextran are dissolved in 12.5 µL of enzyme premix in Embodiment 1, so that a volume of each reaction is increased from 12.5 µL to 13.5 µL after the sugars are added. An effect of the sugars on the enzyme premix is detected by evaluating reaction performance by using a SARS-CoV-2 primer mixture (for the used primer, refer to https://sfanyournals.onlinelibrary.wiley.com/doi/full/10.1111/1751-7915.13586). The left image in FIG. 4 shows systems that are not dispensed before a LAMP reaction (both the systems contain a phenol red indicator, and a color of phenol red changes with a pH value of a solution in which phenol red is located). A control group is a group without the two sugars, and an experimental group is a group added with the two sugars. Colors of the two groups remain unchanged and are red before the reaction. The right image in FIG. 4 shows color development results of two groups of eight-tube strips after a LAMP amplification reaction (heating for 30 minutes at 65°C) is performed on the control group and the experimental group. The results indicate that the color development results of the control group and the experimental group are the same, which indicates that the system containing the enzyme and the sugars and the system without the sugars have no obvious color change after amplification. Therefore, determining may be directly performed based on the color development results after the sugars are added, and the pH value does not need to be additionally adjusted. Each reaction in each eight-tube strip contains a spiked sample with 5000 copies of a Twist SARS-CoV-2 viral RNA and indicates a positive result (four tubes on the left are yellow, and color development degrees of the control group and the experimental group are basically the same), and a negative control without RNase water indicates a negative result (four tubes on the right are red, and color development degrees of the control group and the experimental group are basically the same). A red sample tube indicates that a system contains a limited number of nucleic acid copies, and a yellow sample tube indicates that a system contains a large number of newly amplified nucleic acid copies.

### Embodiment 4 Sensitivity Detection

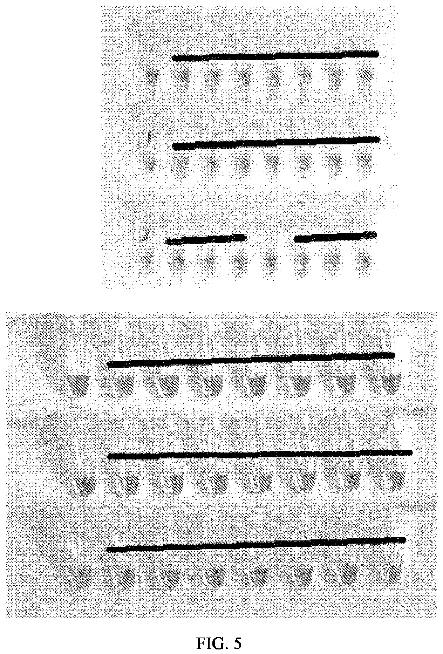

The enzyme pellets obtained in Embodiment 1 are added to a LAMP reaction system containing a COVID-19 primer (https://sfamjoumals.onlinelibrary.wiley.com/doi/full/10.1111/1751-7915.13586) to perform a LAMP amplification reaction. FIG. 5 shows sensitivity comparison between a LAMP enzyme pellet (in the lower image) and a current lyophilized LAMP product (Oxford MEStar, Oxsed Ravid SARS-CoV-2 test, in the upper image). These tests are performed by using a COVID-19 primer mixture and adding different numbers of copies of a Twist viral RNA. In the upper image, 0, 10, 20, 40, 80, 100, 200, and 2000 copies are sequentially added to 25 µL reaction systems in an eight-tube strip respectively from the left to the right. In the lower image, 0, 25, 50, 75, 100, 200, 400, and 1000 copies are sequentially added to 25 µL reaction systems in an eight-tube strip respectively from the left to the right.

In FIG. 5, systems in sample tubes at black horizontal lines are yellow. A result in the lower image in FIG. 5 indicates that 25 copies of a spiked Twist RNA can be always detected in each 25 µL reaction in a mixed system of the enzyme pellet and the COVID-19 primer. Consistent positive results for the 25 copies in each of the 25 µL reactions indicate that the enzyme pellets prepared according to the method in the present disclosure are functional and that the method can be used to implement large-scale production of a lyophilized LAMP product.

### Embodiment 5

Sensitivity of LAMP detection is analyzed based on seven different sugar concentrations (Table 7). The experiment involves two types of sugars: trehalose as a cryopreservation agent and dextran as a filler. The sensitivity of LAMP detection is studied by using three types of Twist RNAs with different concentrations (100, 500, and 1000 viral RNA copies/reaction). A detection method is the same as that in Embodiment 4.

**Table 7: Seven trehalose concentration-dextran concentration groups**

| Group | 1 | 2 | 3 | 4 | 5 | 6 | 7 |
|---|---|---|---|---|---|---|---|
| Trehalose | 9% | 9% | 9% | 9% | 5% | 15% | 20% |
| Dextran | 9% | 1% | 5% | 15% | 9% | 9% | 9% |

| | | | | | | | |
|---|---|---|---|---|---|---|---|
| Note: A percentage represents a mass ratio of a sugar to an enzyme premix. | | | | | | | |

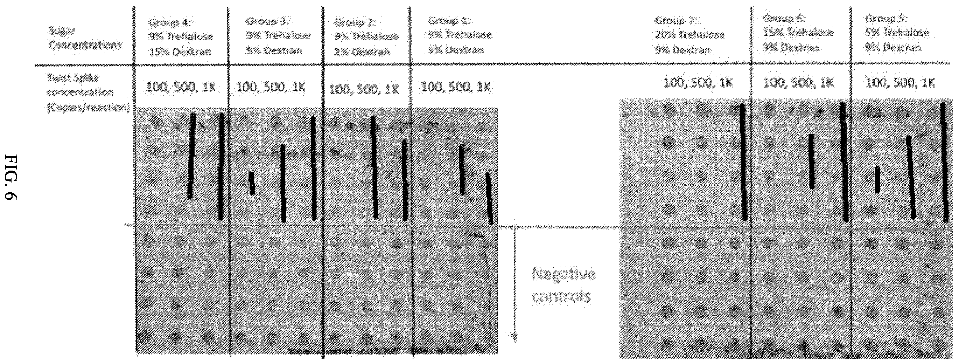

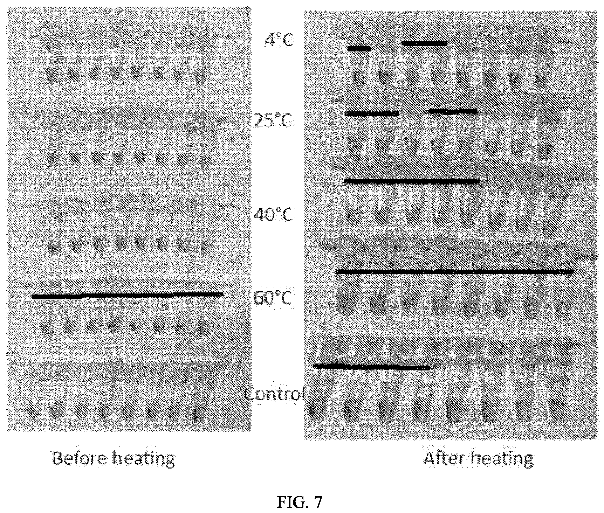

FIG. 7 shows color development results obtained after a LAMP amplification reaction is performed by using lyophilized enzyme products with different sugar concentrations. The results indicate the following cases: Sensitivities of LAMP detection can be high when the trehalose concentrations are at a same level (9%) and the dextran concentrations are below 9%, in particular, are 1% to 5%. The sensitivities basically reach a same standard when the dextran concentrations are at a same level (9%) and the trehalose concentrations are below 20%, and each reaction can reach at least about 1000 copies (FIG. 6). In particular, the sensitivities of LAMP detection are higher when the trehalose concentrations are 5% to 15%. Therefore, when the trehalose concentration is set to be 5% to 20%, preferably, 5% to 15%, and the dextran concentration is set to be 1% to 9%, preferably, 1% to 5%, high sensitivity can be obtained by using the enzyme pellet system of the present disclosure.

### Embodiment 6 Preservation of an Enzyme pellet at Room Temperature

The enzyme pellets obtained in Embodiment 1 of the present disclosure are separately preserved at 4°C, 25°C, 40°C, and 60°C for 7 days, a LAMP amplification reaction is performed by using a same method as that in Embodiment 4, and statistics collection is performed on color changes between before heating and after heating. Results in FIG. 7 (the left image shows the ones before amplification and the right image shows the ones after a LAMP amplification reaction) indicate the following cases: The enzyme pellets provided in the present disclosure cannot be preserved at 60°C (after being preserved at 60°C, sample tubes are yellow before and after a reaction, and whether a virus exists in the sample tubes cannot be determined through color development). However, after being preserved at 4°C, 25°C, and 40°C, sample tubes are slightly changed into orange after the reaction, which indicates that the enzyme pellets of the present disclosure are still active after being preserved at 4°C, 25°C, and 40°C for 7 days, and can still be used in the LAMP amplification reaction for detecting existence of a virus. Each reaction is added with 500 viral RNA copies/reaction. Sample tubes at black lines in FIG. 7 are yellow or orange.

In the description of this specification, reference terms such as "an embodiment", "some embodiments", "example", "specific example", or "some examples" mean that specific features, structures, materials, or characteristics described with reference to the embodiments or examples are included in at least one embodiment or example of the present disclosure. In this specification, the example expressions of the previous terms are not necessarily with respect to the same embodiments or examples. In addition, the described specific features, structures, materials, or characteristics can be combined in a proper manner in any one or more of the embodiments or examples. In addition, a person skilled in the art can integrate and combine different embodiments or examples and features in different embodiments or examples described in this specification, provided that they do not conflict with each other.

Although the embodiments of the present disclosure have been shown and described above, it can be understood that the above-mentioned embodiments are exemplary and cannot be understood as limiting the present disclosure, and a person of ordinary skill in the art can make changes, modifications, replacements, and variations to the above-mentioned embodiments within the scope of the present disclosure.

## Claims

1. An enzyme composition capable of being preserved at room temperature, wherein the enzyme composition comprises:
an enzyme;
an enzyme stabilizer; and
a filler;
the enzyme stabilizer is selected from optional sucrose and at least one of trehalose or trehalose dihydrate; and
the filler is selected from at least one of dextran, mannitol, polyethylene glycol, bovine serum albumin, polyvinyl alcohol, raffinose, or sorbitol.

2. The enzyme composition according to claim 1, wherein a weight ratio of the enzyme stabilizer to the enzyme is (5-20):100, and a weight ratio of the filler to the enzyme is (1-15): 100.

3. The enzyme composition according to claim 1 or 2, wherein the enzyme composition is a lyophilized product obtained through lyophilization.

4. The enzyme composition according to claim 3, wherein the lyophilized product is lyophilized powder or a lyophilized pellet.

5. The enzyme composition according to any one of claims 1 to 4, wherein the enzyme composition is a pellet, and a diameter of the pellet is 0.1 mm to 20 mm.

6. The enzyme composition according to any one of claims 1 to 5, wherein the enzyme in the enzyme composition comprises at least one of a DNA polymerase, an RNA polymerase, a reverse transcriptase, a restriction endonuclease, a recombinase, a ligase, a hydrolase, a helicase, a cellulase, a pectinase, a protease, or a lipase.

7. The enzyme composition according to any one of claims 1 to 6, wherein the enzyme in the enzyme composition comprises at least one of the DNA polymerase, the RNA polymerase, the reverse transcriptase, or the recombinase.

8. A method for preparing the enzyme composition according to any one of claims 1 to 7, wherein the method comprises:
(1) preparing an enzyme-containing preparation;
(2) mixing the enzyme-containing preparation with the enzyme stabilizer and the filler to obtain a to-be-lyophilized mixture; and
(3) lyophilize the to-be-lyophilized mixture to obtain the enzyme composition capable of being preserved at room temperature.

9. The method according to claim 8, wherein an enzyme in the enzyme-containing preparation is a mixture of a DNA polymerase and a reverse transcriptase.

10. The method according to claim 8 or 9, wherein the enzyme stabilizer is trehalose dihydrate.

11. The method according to any one of claims 8 to 10, wherein the filler is dextran.

12. The method according to any one of claims 9 to 11, wherein a concentration of the enzyme is 0.1 U/µL to 5.0 U/µL.

13. The method according to any one of claims 9 to 12, wherein a weight ratio of the enzyme stabilizer to the enzyme is (5-20):100.

14. The method according to any one of claims 9 to 13, wherein a weight ratio of the filler to the enzyme is (1-15):100.

15. The method according to any one of claims 8 to 14, wherein the lyophilization is implemented by the following steps:
1) performing liquid nitrogen freezing on the to-be-lyophilized mixture to obtain a to-be-lyophilized product; and
2) performing vacuum lyophilization on the to-be-lyophilized product at -35°C to -80°C to obtain the enzyme composition capable of being preserved at room temperature; and
a time for the vacuum lyophilization is at least 35 h.

16. The method according to claim 15, wherein a temperature for the vacuum lyophilization is -35°C to -60°C.

17. The method according to claim 15, wherein a temperature for the vacuum lyophilization is -40°C to -45°C.

18. The method according to any one of claims 8 to 17, wherein the room temperature is 20°C to 40°C.

19. A use of the enzyme composition capable of being preserved at room temperature according to any one of claims 1 to 7 or the enzyme composition prepared according to the method according to any one of claims 8 to 18 in preparing a kit, wherein the kit is used for detecting a target nucleic acid.

20. The use according to claim 19, wherein the enzyme contained in the enzyme composition comprises at least one of a DNA polymerase, an RNA polymerase, or a reverse transcriptase.

21. The use according to claim 19 or 20, wherein a nucleic acid is a DNA or an RNA.

22. A kit, wherein the kit contains the enzyme composition capable of being preserved at room temperature according to any one of claims 1 to 7 or the enzyme composition prepared according to the method according to any one of claims 8 to 18.

23. The kit according to claim 22, wherein the enzyme contained in the enzyme composition comprises at least one of a DNA polymerase, an RNA polymerase, or a reverse transcriptase.

24. A nucleic acid amplification reaction system, wherein the nucleic acid amplification reaction system comprises the enzyme composition capable of being preserved at room temperature according to any one of claims 1 to 7 or the enzyme composition prepared according to the method according to any one of claims 8 to 18.

25. The nucleic acid amplification reaction system according to claim 24, wherein the nucleic acid amplification reaction system further comprises a primer, dNTP, and a reaction buffer for nucleic acid amplification.

26. The nucleic acid amplification reaction system according to claim 24 or 25, wherein the nucleic acid amplification reaction system further comprises a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

27. The nucleic acid amplification reaction system according to claim 26, wherein the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

28. The nucleic acid amplification reaction system according to claim 26, wherein the fungus is selected from at least one of yeast, mold, candida albicans, cryptococcus, aspergillus, or mucor, and the chlamydia is chlamydia trachomatis.

29. The nucleic acid amplification reaction system according to claim 26, wherein the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

30. The nucleic acid amplification reaction system according to claim 29, wherein the coronavirus is selected from at least one of SARS-CoV, SARS-CoV-2, SARS-CoV-19, MERS-CoV, HCoV-229E, HCoV-OC43, HCoV-NL63, or HCoV-HKU1.

31. The nucleic acid amplification reaction system according to claim 29, wherein the influenza virus is selected from influenza A virus and influenza B virus.

32. The nucleic acid amplification reaction system according to claim 26, wherein the protozoan is selected from at least one of plasmodium, trichomonas vaginalis, toxoplasma gondii, leishmania, babesia, entamoeba histolytica, acanthamoeba, or trypanosoma brucei.

33. A LAMP reaction system, wherein the LAMP reaction system comprises the enzyme composition capable of being preserved at room temperature according to any one of claims 1 to 7 or the enzyme composition prepared according to the method according to any one of claims 8 to 18.

34. The LAMP reaction system according to claim 33, wherein the enzyme in the enzyme composition is a DNA polymerase or a mixture of a DNA polymerase and a reverse transcriptase.

35. The LAMP reaction system according to claim 34, wherein the DNA polymerase is a Bst DNA polymerase.

36. The LAMP reaction system according to any one of claims 33 to 35, wherein the LAMP reaction system further comprises a primer, dNTP, and a reaction buffer for nucleic acid amplification.

37. The LAMP reaction system according to any one of claims 33 to 36, wherein the LAMP reaction system further comprises a to-be-detected sample, and the to-be-detected sample is suspected to contain at least one of bacterial cells or a culture fluid of a bacterium, fungal cells or a culture fluid of a fungus, chlamydia, treponema pallidum, a virus, a DNA isolated from a protozoan, or an RNA isolated from a protozoan.

38. The LAMP reaction system according to claim 37, wherein the bacterium is selected from at least one of escherichia coli, agrobacterium tumerfaciens, staphylococcus aureus, streptococcus, coagulase negative staphylococcus, clostridium difficile, bacillus anthraci, enterococcus, corynebacteria, mycobacteria, pseudomonas aeruginosa, salmonella, neisseria gonorrhoeae, listeria monocytogenes, leptospira, burgdorferi, campylobacter, brucella, vibrio cholerae, or yersinia pestis.

39. The LAMP reaction system according to claim 37, wherein the fungus is selected from at least one of yeast, mold, candida albicans, cryptococcus, aspergillus, or mucor, and the chlamydia is chlamydia trachomatis.

40. The LAMP reaction system according to claim 37, wherein the virus is selected from at least one of coronavirus, influenza virus, Nipah virus, hepatitis B virus, hepatitis A virus, human immunodeficiency virus, rabies virus, dengue fever virus, respiratory syncytial virus, Ebola virus, human papilloma virus, herpes virus, paramyxovirus, Chikungunya virus, Japanese encephalitis virus, Zika virus, West Nile virus, Marburg virus, or yellow fever virus.

41. The LAMP reaction system according to claim 40, wherein the coronavirus is selected from at least one of SARS-CoV, SARS-CoV-2, SARS-CoV-19, MERS-CoV, HCoV-229E, HCoV-OC43, HCoV-NL63, or HCoV-HKU1.

42. The LAMP reaction system according to claim 40, wherein the influenza virus is selected from influenza A virus and influenza B virus.

43. The LAMP reaction system according to claim 37, wherein the protozoan is selected from at least one of plasmodium, trichomonas vaginalis, toxoplasma gondii, leishmania, babesia, entamoeba histolytica, acanthamoeba, or trypanosoma brucei.

44. A method for detecting a target nucleic acid in a to-be-detected sample, wherein the method comprises:
(i). obtaining the to-be-detected sample or obtaining a nucleic acid isolated from the to-be-detected sample;
(ii). using the to-be-detected sample or the nucleic acid as a template, and amplifying the template by using a specific primer for the target nucleic acid and using the enzyme composition capable of being preserved at room temperature according to any one of claims 1 to 7 or the enzyme composition prepared according to the method according to any one of claims 8 to 18, to obtain an amplified product; and
(iii). detecting whether the amplified product contains a plurality of copies of the target nucleic acid, wherein if the amplified product contains the plurality of copies of the target nucleic acid, it indicates that the to-be-detected sample contains the target nucleic acid.

45. The method according to claim 44, wherein the method further comprises:
performing sequencing on the amplified product to obtain a nucleic acid sequence contained in the amplified product, and comparing the nucleic acid sequence with a reference sequence to determine whether a mutation occurs on the target nucleic acid contained in the to-be-detected sample.

46. The method according to claim 45, wherein the reference sequence is a nucleic acid sequence of a wild-type target nucleic acid that is not mutated.

47. The method according to any one of claims 44 to 46, wherein the nucleic acid is a DNA or an RNA.

48. The method according to any one of claims 44 to 47, wherein the to-be-detected sample is derived from a plant, an animal, or an environment sample.

49. The method according to claim 48, wherein the animal is a mammal, and the mammal is selected from a human, a rat, a mouse, a pig, cattle, a sheep, a dog, and a cat.
